# EUROPEAN PATENT SPECIFICATION

(11) **EP 2 474 263 B1**
(45) Date of publication and mention of the grant of the patent: **22.05.2013**
(21) Application number: 12161506.6
(22) Date of filing: 24.03.2010
(51) Int. Cl.: A61B 5/0478

(54) **Attaching device for holding dry electrodes for detecting EEG signals**
Befestigungsvorrichtung zum Halten von Trockenelektroden zur EEG-Signalerfassung
Dispositif d'attache pour fixer des électrodes à sec pour détecter les signaux EEG

(43) Date of publication of application: 11.07.2012
(62) Divisional of application: 10157605.6
(73) Proprietor: Brain Products GmbH, 82205 Gilching (DE)
(72) Inventor: Picht, Bernd, 82110 Germering (DE); Svojanovsky, Alexander, 82205 Gilching (DE)
(74) Representative: TBK

(56) References cited:
- WO-A1-2008/127620
- WO-A2-2008/017998
- US-A- 2 426 958
- US-B1- 6 574 513

## Description

The present invention relates to an electrode for detecting EEG signals and an attaching device for holding the electrode which allow contacting the scalp without a conductive gel between the scalp and the electrode.

In order to provide contact between an electrode used for measuring EEG (Electro-Encephalography) signals and the scalp, usually a conductive gel is applied between the skin and the electrode.

However, applying the gel is cumbersome and dry electrodes are aimed for which do not require a conductive gel.

The US 6 574 513 B1 discloses an EEG electrode system for establishing positive electrical contact with the scalp, which uses absorptive electrode wrap material in contact with metallic electrodes, with saturated electrode wrap material placed in contact with the scalp. Saturation of the electrode warp material with a conductive solution such as saline provides mechanically and electrically stable contact at multiple scalp electrode sites. An electrode wrap holder assembly holds the electrode wrap and an exposed portion of the electrode wrap in contact with the scalp, while providing access to the electrode wrap for re-wetting with the conductive solution if required. The electrode wrap holders are mounted in straps, hard caps or other headgear. Adjustment of the electrode wrap holders within the mounts further compresses the flexible, absorptive electrode wrap, causing flow of the conductive solution therein and enhancement of the electrical contact.

The US 2 426 9598 A discloses a device for applying and holding electrodes in electrical contact with the scalp for recording electrical potentials of cortical areas.

The WO 2008/017998 A2 discloses an ultrasonic diagnostic imaging system which utilizes one or more transducer arrays affixed to the head of a patient to diagnose and treat stroke victims. The transducer headset produces a two or three dimensional image of the vasculature inside the cranium, preferably assisted by a microbubble contrast agent. A vascular flow map is produced by the system which may be diagnosed for signs of a blood clot. If a blood clot is detected, a therapeutic beam is transmitted while the contrast agent is present to break up the blood clot by the disruption of microbubbles. The headset may also be used in a monitoring application to detect the recurrence of blood clots in a stroke victim.

The WO 2008/127620 A1 discloses a portable apparatus having a source, a detector and a filtering element placed a known distance from the source and/or the detector. One example of the apparatus is a headlamp having an illumination source, a camera, and two eye pieces. The headlamp has an adjustable headband for positioning the headlamp on the head of the user and a protective cover for interacting with the camera, eye pieces, and/or illumination source. The protective cover preferably includes multiple sets of filtering elements such that motion about or along an axis changes the filtering elements interacting with the camera, eye pieces, and/or the illumination source.

In particular, the present invention aims at providing a dry electrode for detecting EEG signals and an attaching device for holding the dry electrode which serve to ensure contact between the dry electrode and the scalp through the scalp hair.

In the following the invention will be described by way of embodiments thereof with reference to the accompanying drawings, in which:
Fig. 1 shows a dry electrode according to an embodiment of the invention;
Fig. 2 shows an attaching device for holding dry electrodes according to an embodiment of the invention; and
Fig. 3 shows a first surface of the rotary closure of the attaching device, according to an embodiment of the invention.

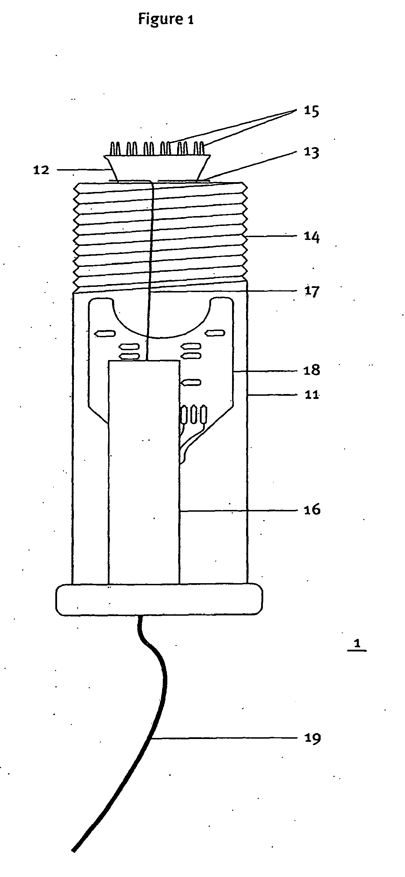

Referring to Fig. 1, an electrode 1 for detecting EEG signals is shown which comprises a body 11 and a sensor 12. The sensor 12 is detachably held by a fixture 13 of the body 11. The fixture 13 is provided on a first end of the body 11, which is on the side of the scalp when the electrode 1 is attached for detecting EEG signals. In other words, the fixture 13 is formed on a plane of the body 11, which faces the scalp when the electrode 1 is attached for detecting EEG signals. When detecting EEG signals, the sensor 12 is in contact with the scalp and senses an electrical potential at its place on the scalp.

The body 11 may be of cylindrical shape and has an external thread 14 in a portion of the body 11 extending from the first end of the body 11 towards a second end of the body 11, which is off the scalp when the electrode 1 is attached for detecting EEG signals. The external thread 14 may be provided in the first third of the body 11 with respect to a direction from the first end to the second end of the body 11. The external thread 14 serves to attach or fix the electrode 1 in a hole of a strap of an attaching device to be described later, and to adjust the distance between the sensor 12 and the scalp.

The sensor 12 has a brush-like shape with pins 15 protruding off the body 11 and towards the scalp through the scalp hair when the electrode 1 is attached for detecting EEG signals. The sensor 12 is made of Ag, AgCl, Au, Sn or any other conductive material including conductive polymer.

The fixture 13 may be a clip into which the sensor 12 can be clicked and from which the sensor 12 can be detached. This arrangement allows an exchange of the sensor in order to replace the sensor, clean the sensor, etc.

According to an example, the body 11 may include a vibration element 16 and a connector 17 which transfers a vibration of the vibration element 16 to the sensor 12. The connector 17 may be a rod or the like which is clamped between the vibration element 16 and the sensor 12. By means of the vibration, the sensor 12, in particular the pins 15 of the sensor 12, is/are moved through hair of the scalp, when the electrode 1 is attached for detecting EEG signals. The vibration element 16 may be attached to the second end of the body 11 and may extend in the body 11 towards the first end of the body 11, as shown in Fig. 1. The vibration element 16 may comprise an element including a vibration motor, such as an element as e.g. used in cell phones.

It is to be noted that the vibration element 16 may be arranged at any position in the electrode 1 as long as the vibration of the vibration element can be transferred to the sensor 12 to cause the sensor 12 to vibrate through the scalp hair. The vibration element may also be used as a feedback instrument allowing an inference on an electrical potential sensed by the sensor 12.

The body 11 includes a circuit board 18 which is casted watertight and comprises circuitry for impedance conversion of an electrical potential sensed by the sensor 12 and input to the circuitry and for impedance display. The impedance display may be arranged by LEDs of different colors.

The electrode 1 has a lead 19 which emanates from the second end of the body 11 and receives from the circuit board 18 a signal representing the electrical potential sensed by the sensor 12 and impedance converted by the circuitry of the circuit board 18 and outputs the signal from the electrode 19 for further processing of the signal. The lead 19 may also be used for transferring power to the circuit board 18 and the vibration element 16.

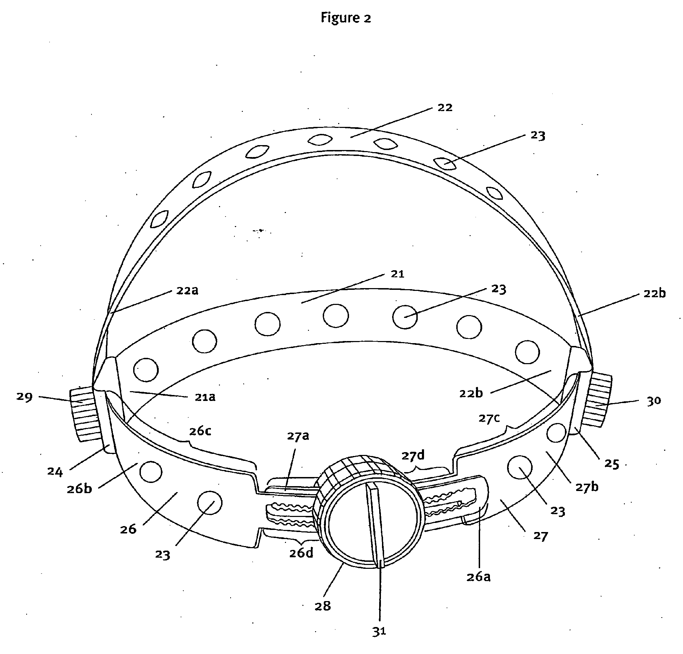

Fig. 2 shows an embodiment of an attaching device 2 for holding electrodes for detecting EEG signals, such as the electrode 1 shown in Fig. 1. When used for detecting EEG signals, the attaching device 2 is placed on a head. According to an embodiment of the invention, the attaching device 2 comprises a first strap 21 including a plurality of holes 23 for accommodating electrodes. As shown in Fig. 2, the attaching device 2 may further comprise a second strap 22 including a plurality of holes 23 for accommodating electrodes. A first joint 24 connects a first end 21a of the first strap 21 and a first end 22a of the second strap 22, and a second joint 25 connects a second end 21b of the first strap 21 and a second end 22b of the second strap 22. The second joint 25 is disposed opposite to the first joint 24.

It is to be noted that the attaching device 2 may comprise several second straps 22 in order to provide for further electrode positions. In case of several second straps 22, first ends 22a of the second straps are connected to the first joint 24, and second ends 22b of the second straps 22 are connected to the second joint 25.

The attaching device 2 further includes a third strap 26, a fourth strap 27 and a rotary closure 28 which connects a first end 26a of the third strap 26 and a first end 27a of the fourth strap 27. A second end 26b of the third strap 26 is connected to the first joint 24 and a second end 27b of the fourth strap 27 is connected to the second joint 25. As shown in Fig. 2, the first, third and fourth straps may be connected to form a ring, and, if present, the second strap may be connected to form an arc over the ring. In case of several second straps 22, these may also be connected to from an arc over the ring.

The third and fourth straps 26, 27 may also include holes 23 for accommodating electrodes in a portion 26c, 27c extending from the second end 26b, 27b towards the first end 26a, 27a, as shown in Fig. 2.

Into the holes 23 electrodes such as the electrode 1 may be placed/screwed so that different lead positions are possible.

According to an embodiment of the invention, the first and second joints 24, 25 may comprise fixing screws 29, 30 allowing fixation and change of the first to fourth straps 21, 22, 26 and 27 in order to adjust the attaching device to different head sizes. Straps of different lengths may be used for the first to fourth straps in order to pattern the form of a head. The first ends 21a, 22a of the first and second straps 21, 22 and the second end 26b of the third strap 26 are inserted into the joint 24 and fixed by the fixing screw 29, and the second ends 21b, 22b of the first and second straps 21, 22 and the second end 27b of the fourth strap 27 are inserted into the joint 25 and fixed by the fixing screw 30, allowing nearly free positioning of the first and second straps and, thus, of electrodes on a head which are inserted into the holes 23.

According to an embodiment of the invention in which the attaching device 2 does not include any second straps 22, the first and second joints 24, 25 and the fixing screws 29, 30 may be omitted, and the first, third and fourth straps may be formed as one strap.

At least one of the first to fourth straps 21, 22, 26, 27 may be made of flexible and/or stretchable material in order to improve adjustment of the attaching device 2 to different head sizes. The first to fourth straps 21, 22, 26 and 27 may be made of polyurethane (PUR).

The third strap 26 and the fourth strap 27 each have a portion 26d, 27d which extends from the first end 26a, 27a towards the second end 26b, 27b and is formed as a rack-like closure aid. The rotary closure 28 includes an actuator (not shown) cogging the rack-like closure aid and an operating element 31 at a second surface of the rotary closure 28, which connects to the actuator, so that a ring formed by the third and fourth straps 26, 27 and the first strap 21 narrows or widens when the operating element 31 is operated by a user of the attaching device 2. That is, when the attaching device 2 is placed on a head, by rotating the operating element 31 the first strap 21 and the third and fourth straps 26, 27 are fastened on the head or released depending on the rotation direction. Thus, contact pressure of electrodes, in particular of the pins 15 of electrode 1, inserted into the holes 23 can be ensured.

The rack-like closure aid may be a flexible rack, preferably having two rows of teeth placed inside and facing each other as shown in Fig. 2, and the actuator may by a rack-wheel.

According to an embodiment of the invention in which the attaching device does not include any second straps 22, the attaching device comprises a strap including a plurality of holes for accommodating electrodes, e.g. the first, third and fourth straps shown in Fig. 2 formed as one strap. The attaching device further comprises a rotary closure, such as the rotary closure 28, connecting a first end of the strap and a second end of the strap. The first end of the strap may be similar to the first end of the third strap 26 shown in Fig. 2, and the second end of the strap may be similar to the first end of the fourth strap 27 shown in Fig. 2. The strap may have a first portion at the first end and a second portion at the second end, wherein the first and second portions are formed as a rack-like closure aid. The first portion may be similar to the portion 26d of the third strap shown in Fig. 2, and the second portion may be similar to the portion 27d of the fourth strap shown in Fig. 2. The strap may be made of flexible material.

The attaching device with the above arrangements can be easily fit and adjusted to each head shape. Once the attaching device has been fit and adjusted to a person's head, re-fitting the attaching device to the person's head can be done even quicker.

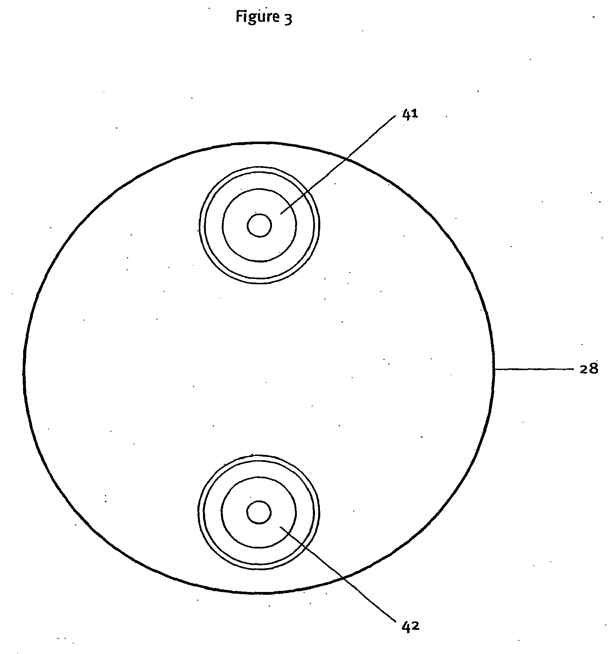

Referring to Fig. 3 showing a plan view of a first surface of the rotary closure 28, the rotary closure 28 includes a reference electrode 41 and a ground electrode 42 which are arranged centrally and on top of each other on the first surface of the rotary closure 28. The reference electrode 41 and the ground electrode 42 are in contact with a forehead when the attaching device 2 is placed on a head and are positioned substantially centrally and on top of each other on a center line of the head. When the attaching device 2 is placed on the head it is substantially symmetric to a plane passing through the center line of the head and bisecting the head.

The first surface of the rotary closure 28 shown in Fig. 3 is opposite to the second surface of the rotary closure 28 shown in Fig. 2.

The reference electrode 41 and the ground electrode 42 may be designed as cup electrodes or pads.

The rotary closure 28 may have a round shape as shown in Fig. 3, but is not limited thereto.

It is to be understood that the above description is illustrative of the invention and is not to be construed as limiting the invention. Various modifications and applications may occur to those skilled in the art without departing from the scope of the invention as defined by the appended claims.

This application is a divisional application of European patent application no. 10 157 605.6 (the "parent application"), also published as EP 2 368 494. Based on the original claims of the parent application, the following aspects form part of the content of this divisional application as filed.
1. An electrode for detecting EEG signals, comprising:
   a body comprising a fixture on a first end of the body; and
   a sensor detachably held by the fixture,
   wherein the body has an external thread in a portion of the body extending from the first end of the body towards a second end of the body, and
   the sensor has a brush-like shape with pins protruding off the body.
2. The electrode of aspect 1, wherein the body includes a vibration element and a connector which transfers a vibration of the vibration element to the sensor.
3. The electrode of aspect 1 or 2, wherein the body includes a circuit board which is casted watertight and comprises circuitry for impedance conversion of an electrical potential sensed by the sensor and for impedance display.
4. An attaching device for holding electrodes for detecting EEG signals, the attaching device comprising:
   a strap including a plurality of holes for accommodating electrodes; and
   a rotary closure connecting a first end of the strap and a second end of the strap.
5. The attaching device of aspect 4, wherein the strap has a first portion at the first end and a second portion at the second end, wherein the first and second portions are formed as a rack-like closure aid, and the rotary closure includes an actuator cogging the rack-like closure aid and an operating element at a second surface of the rotary closure, which connects to the actuator so that a ring formed by the strap narrows or widens when the operating element is operated.
6. The attaching device of aspect 4 or 5, wherein the strap is made of flexible material.
7. An attaching device for holding electrodes for detecting EEG signals, the attaching device comprising:
   a first strap and at least one second strap each including a plurality of holes for accommodating electrodes;
   a first joint connecting a first end of the first strap and a first end of the at least one second strap, and a second joint connecting a second end of the first strap and a second end of the at least one second strap;
   a third strap and a fourth strap; and
   a rotary closure connecting a first end of the third strap and a first end of the fourth strap,
   wherein a second end of the third strap is connected to the first joint and a second end of the fourth strap is connected to the second joint.
8. The attaching device of aspect 4 or 7, wherein the rotary closure includes a reference electrode and a ground electrode which are arranged centrally and on top of each other on a first surface of the rotary closure, wherein the reference electrode and the ground electrode are in contact with a forehead when the attaching device is placed on a head and are positioned substantially centrally and on top of each other on a center line of the head.
9. The attaching device of aspect 7 or 8, wherein the third strap and the fourth strap each have a portion which extends from the first end towards the second end and is formed as a rack-like closure aid, and the rotary closure includes an actuator cogging the rack-like closure aid and an operating element at a second surface of the rotary closure, which connects to the actuator so that a ring formed by the third and fourth straps and the first strap narrows or widens when the operating element is operated.
10. The attaching device of any one of aspects 7 to 9, wherein the first and second joints comprise fixing screws.
11. The attaching device of any one of aspects 7 to 10, wherein the third and fourth straps include holes for accommodating electrodes in a portion extending from the second end towards the first end.
12. The attaching device of any one of aspects 7 to 11, wherein at least one of the first to fourth straps are made of flexible material.

## Claims

1. An attaching device for holding electrodes for detecting EEG signals, the attaching device comprising:
a strap (21, 26, 27) including a plurality of holes (23) for accommodating electrodes; and
a rotary closure (28) connecting a first end of the strap and a second end of the strap,
**characterized in that**
the rotary closure (28) includes a reference electrode (41) and a ground electrode (42) which are arranged on a first surface of the rotary closure, wherein the reference electrode and the ground electrode are adapted to be in contact with the forehead and to be positioned substantially centrally and spaced apart on a center line of a head when the attaching device is placed on the head.

2. The attaching device of claim 1, wherein the strap has a first portion at the first end and a second portion at the second end, wherein the first and second portions are formed as a rack-like closure aid, and the rotary closure includes an actuator cogging the rack-like closure aid and an operating element (31) at a second surface of the rotary closure, which connects to the actuator so that a ring formed by the strap narrows or widens when the operating element is operated.

3. The attaching device of claim 1 or 2, wherein the strap is made of flexible material.

4. The attaching device of claim 1, wherein the strap comprises a first strap (21), a third strap (26) and a fourth strap (27), the first strap including a plurality of holes for accommodating electrodes, the attaching device further comprising:
at least one second strap (22) including a plurality of holes for accommodating electrodes; and
a first joint (24) connecting a first end (21a) of the first strap (21) and a first end (22a) of the at least one second strap (22), and a second joint (25) connecting a second end (21b) of the first strap (21) and a second end (22b) of the at least one second strap (22),
wherein the rotary closure (28) connects a first end (26a) of the third strap (26) and a first end (27a) of the fourth strap (27),
wherein a second end (26b) of the third strap (26) is connected to the first joint (24) and a second end (27b) of the fourth strap (27) is connected to the second joint (25).

5. The attaching device of claim 4, wherein the third strap (26) and the fourth strap (27) each have a portion (26d, 27d) which extends from the first end towards the second end and is formed as a rack-like closure aid, and the rotary closure (28) includes an actuator cogging the rack-like closure aid and an operating element at a second surface of the rotary closure, which connects to the actuator so that a ring formed by the third and fourth straps and the first strap narrows or widens when the operating element (31) is operated.

6. The attaching device of claim 4 or 5, wherein the first and second joints comprise fixing screws (29, 30).

7. The attaching device of any one of claims 4 to 6, wherein the third and fourth straps include holes for accommodating electrodes in a portion extending from the second end towards the first end.

8. The attaching device of any one of claims 4 to 7, wherein at least one of the first to fourth straps are made of flexible material.

## Patentansprüche

1. Anbringungsvorrichtung zum Halten von Elektroden zur Erfassung von EEG-Signalen, wobei die Anbringungsvorrichtung umfasst:
ein Band (21, 26, 27) mit einer Vielzahl von Löchern (23) zum Aufnehmen von Elektroden und
einen Drehverschluss (28), der ein erstes Ende des Bandes und ein zweites Ende des Bandes verbindet,
**dadurch gekennzeichnet, dass**
der Drehverschluss (28) eine Referenzelektrode (41) und eine Masseelektrode (42) enthält, die an einer ersten Oberfläche des Drehverschlusses angeordnet sind, wobei die Referenzelektrode und die Masseelektrode dazu eingerichtet sind, mit der Stirn in Kontakt zu sein und im Wesentlichen mittig und beabstandet auf einer Mittellinie eines Kopfes positioniert zu sein, wenn die Anbringungsvorrichtung auf dem Kopf platziert ist.

2. Anbringungsvorrichtung nach Anspruch 1, wobei das Band einen ersten Abschnitt an dem ersten Ende und einen zweiten Abschnitt an dem zweiten Ende aufweist, wobei der erste und der zweite Abschnitt als schienenähnliche Verschlusshilfe ausgebildet sind, und der Drehverschluss ein Betätigungselement, das die schienenähnliche Verschlusshilfe verzahnt, und ein Bedienelement (31) an einer zweiten Oberfläche des Drehverschlusses enthält, das mit dem Betätigungselement derart verbunden ist, dass ein durch das Band gebildeter Ring sich bei Betätigung des Bedienelements verengt oder erweitert.

3. Anbringungsvorrichtung nach Anspruch 1 oder 2, wobei das Band aus flexiblem Material gebildet ist.

4. Anbringungsvorrichtung nach Anspruch 1, wobei das Band ein erstes Band (21), ein drittes Band (26) und ein viertes Band (27) umfasst, wobei das erste Band eine Vielzahl von Löchern zur Aufnahme von Elektroden enthält, wobei die Anbringungsvorrichtung ferner umfasst
zumindest ein zweites Band (22), das eine Vielzahl von Löchern zur Aufnahme von Elektroden enthält, und
ein erstes Verbindungselement (24), das ein erstes Ende (21a) des ersten Bandes (21) und ein erstes Ende (22a) des zumindest einen zweiten Bandes (22) verbindet, und ein zweites Verbindungselement (25), das ein zweites Ende (21b) des ersten Bandes (21) und ein zweites Ende (22b) des zumindest einen zweiten Bandes (22) verbindet,
wobei der Drehverschluss (28) ein erstes Ende (26a) des dritten Bandes (26) und ein erstes Ende (27a) des vierten Bandes (27) verbindet,
wobei ein zweites Ende (26b) des dritten Bandes (26) mit dem ersten Verbindungselement (24) verbunden ist, und ein zweites Ende (27b) des vierten Bandes (27) mit dem zweiten Verbindungselement (25) verbunden ist.

5. Anbringungsvorrichtung nach Anspruch 4, wobei das dritte Band (26) und das vierte Band (27) jeweils einen Abschnitt (26d, 27d) aufweisen, der sich von dem ersten Ende zu dem zweiten Ende erstreckt und als schienenähnliche Verschlusshilfe ausgebildet ist, und der Drehverschluss (28) ein Betätigungselement, das die schienenähnliche Verschlusshilfe verzahnt, und ein Bedienelement an einer zweiten Oberfläche des Drehverschlusses enthält, das mit dem Betätigungselement derart verbunden ist, dass ein durch das dritte und vierte Band und das erste Band gebildeter Ring sich bei Betätigung des Bedienelements (31) verengt oder erweitert.

6. Anbringungsvorrichtung nach Anspruch 4 oder 5, wobei das erste und das zweite Verbindungselement Fixierschrauben (29, 30) umfassen.

7. Anbringungsvorrichtung nach einem der Ansprüche 4 bis 6, wobei das dritte und vierte Band Löcher zur Aufnahme von Elektroden in einem Abschnitt enthalten, der sich von dem zweiten Ende zu dem ersten Ende erstreckt.

8. Anbringungsvorrichtung nach einem der Ansprüche 4 bis 7, wobei zumindest eines der ersten bis vierten Bänder aus flexiblem Material gebildet ist.

## Revendications

1. Dispositif de fixation destiné à maintenir des électrodes permettant de détecter des signaux EEG, le dispositif de fixation comprenant :
une sangle (21, 26, 27) comportant une pluralité de trous (23) pour recevoir des électrodes ; et
une fermeture rotative (28) reliant une première extrémité de la sangle et une deuxième extrémité de la sangle,
**caractérisé en ce que**
la fermeture rotative (28) comporte une électrode de référence (41) et une électrode de masse (42) qui sont agencées sur une première surface de la fermeture rotative, où l'électrode de référence et l'électrode de masse sont adaptées pour être en contact avec le front et pour être situées essentiellement au centre et espacées sur une ligne centrale d'une tête lorsque le dispositif de fixation est placé sur la tête.

2. Dispositif de fixation de la revendication 1, dans lequel la sangle présente une première partie au niveau de la première extrémité et une deuxième partie au niveau de la deuxième extrémité, où les première et deuxième parties sont formées en tant que moyen auxiliaire de fermeture de type râtelier, et la fermeture rotative comporte un actionneur permettant d'assembler le moyen auxiliaire de fermeture de type râtelier et un élément d'actionnement (31) au niveau d'une deuxième surface de la fermeture rotative, qui est relié à l'actionneur de sorte qu'une bague formée par la sangle se rétrécisse ou s'élargisse lorsque l'élément d'actionnement est actionné.

3. Dispositif de fixation de la revendication 1 ou 2, dans lequel la sangle est réalisée en un matériau souple.

4. Dispositif de fixation de la revendication 1, dans lequel la sangle comprend une première sangle (21), une troisième sangle (26) et une quatrième sangle (27), la première sangle comportant une pluralité de trous pour recevoir des électrodes, le dispositif de fixation comprenant en outre :
au moins une deuxième sangle (22) comportant une pluralité de trous pour recevoir des électrodes ; et
un premier joint (24) reliant une première extrémité (21a) de la première sangle (21) et une première extrémité (22a) de l'au moins une deuxième sangle (22), et un deuxième joint (25) reliant une deuxième extrémité (21b) de la première sangle (21) et une deuxième extrémité (22b) de l'au moins une deuxième sangle (22),
où la fermeture rotative (28) relie une première extrémité (26a) de la troisième sangle (26) et une première extrémité (27a) de la quatrième sangle (27),
où une deuxième extrémité (26b) de la troisième sangle (26) est reliée au premier joint (24) et une deuxième extrémité (27b) de la quatrième sangle (27) est reliée au deuxième joint (25).

5. Dispositif de fixation de la revendication 4, dans lequel chacune de la troisième sangle (26) et la quatrième sangle (27) présente une partie (26d, 27d) qui s'étend depuis la première extrémité vers la deuxième extrémité et est formée en tant que moyen auxiliaire de fermeture de type râtelier, et la fermeture rotative (28) comporte un actionneur permettant d'assembler le moyen auxiliaire de fermeture de type râtelier et un élément d'actionnement au niveau d'une deuxième surface de la fermeture rotative, qui est relié à l'actionneur de sorte qu'une bague formée par les troisième et quatrième sangles et la première sangle se rétrécisse ou s'élargisse lorsque l'élément d'actionnement (31) est actionné.

6. Dispositif de fixation de la revendication 4 ou 5, dans lequel les premier et deuxième joints comprennent des vis de fixation (29, 30).

7. Dispositif de fixation de l'une quelconque des revendications 4 à 6, dans lequel les troisième et quatrième sangles comportent des trous permettant de recevoir des électrodes dans une partie s'étendant depuis la deuxième extrémité vers la première extrémité.

8. Dispositif de fixation de l'une quelconque des revendications 4 à 7, dans lequel au moins une sangle parmi la première sangle à la quatrième sangle est réalisée en un matériau souple.
